# EUROPEAN PATENT SPECIFICATION

(11) **EP 2 222 312 B1**
(45) Date of publication and mention of the grant of the patent: **20.06.2012**
(21) Application number: 08849799.5
(22) Date of filing: 13.11.2008
(51) Int. Cl.: A61K 31/721, A61K 8/37, A61K 33/26, A61P 7/06

(54) **AQUEOUS IRON DEXTRAN PREPARATION HAVING ONE OR MORE PARA-HYDROXYBENZOATE COMPOUNDS AND/OR SALTS THEREOF**
WÄSSRIGE EISEN-DEXTRAN-ZUBEREITUNG MIT EINER ODER MEHREREN PARA-HYDROXYBENZOAT-VERBINDUNGEN UND/ODER SALZEN DAVON
PRÉPARATION AQUEUSE DE FER DEXTRAN RENFERMANT UN OU PLUSIEURS COMPOSÉS PARA-HYDROBENZOATES ET/OU DES SELS DE CEUX-CI

(30) Priority: 13.11.2007 US 987505 P; 13.11.2007 DE 102007054794
(43) Date of publication of application: 01.09.2010
(73) Proprietor: Agilan GmbH, 49377 Vechta (DE)
(72) Inventor: BERGMANN, Stephan, 49377 Vechta (DE)
(74) Representative: Eisenführ, Speiser & Partner
(86) International application number: PCT/EP2008/065496
(87) International publication number: WO 2009/063018

(56) References cited:
- EP-A- 0 044 050
- EP-A- 0 634 174
- WO-A-00/30657
- WO-A-2004/050010
- US-A- 4 623 773

## Description

The present invention relates to an aqueous iron dextran preparation for the prophylaxis or treatment of iron deficiency states in a human or an animal, the iron being present in the form of Fe³⁺ ions and forming a water-soluble complex with the dextran, characterised in that the preparation comprises one, two, three, four or more compounds of the general formula (I) 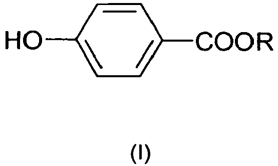 where R represents a C1 to C4 alkyl radical, and/or comprises salts thereof, to a multiple dose preparation for the parenteral prophylaxis or treatment of iron deficiency states in a human or animal, characterised in that the multiple dose preparation comprises a preparation according to the invention and a suitable container, or consists thereof, to a kit comprising or consisting of a) a multiple dose preparation according to the invention and b) syringes suitable for injecting the solution, to a use of the preparation according to the invention for the parenteral prophylaxis or treatment of iron deficiency states in a human or an animal, to a use of one, two, three, four or more compounds of the general formula (I), formula (I) having been described above, to produce an iron dextran preparation according to the invention or to reduce the side effect of an iron dextran preparation according to the invention in the case of parenteral application in a human or an animal, and to a method for producing a preparation according to the invention.

Among anaemias, which are attributable to problems of haemoglobin formation, iron deficiency anaemia has the greatest practical importance, and actually has the greatest importance worldwide as a deficiency disease (Aktories et al., "Allgemeine und spezielle Pharmakologie und Toxikologie" [General and special pharmacology and toxicology], 9th edition, pp. 737 ff., p. 737). Iron deficiency itself can occur because of a low supply usually in combination with restricted bioavailability, or because of blood losses. So that iron can be absorbed from food, trivalent iron (Fe³⁺) is reduced by the ferrireductase Dcytb, which is localized in the brush border membrane of the enterocytes in the small intestine, into divalent iron (Fe²⁺) and is then available to the transport protein DMT1 for transport into the enterocytes.

In order to increase the bioavailability of trivalent iron in the case of oral application from food, it is recommendable to take citric acid or ascorbic acid at the same time since soluble complexes are thereby formed with the trivalent iron in the food and more trivalent iron therefore becomes available for reduction, precisely in the region of the upper small intestinal tract where the trivalent iron precipitates as iron oxyhydrate owing to the prevalent pH, and is therefore no longer available for reduction.

If therapeutic treatment with iron is necessary, iron preparations for oral or parenteral application have now been available for many years.

Iron preparations for oral application are distinguished in that divalent iron is administered directly in the form of its salts, usually in combination with ascorbic acid, fumaric acid, gluconic acid, aspartic acid or with glycine sulphate complex. On the one hand, divalent iron does not precipitate at the pH values in the region of the small intestine where absorption of the iron takes place, and on the other hand divalent iron can be taken up directly by the transport mechanism described above and does not need to be reduced beforehand.

In contrast to this, the parenteral application of trivalent iron uses the fact that it is bound to haemoglobin in this oxidation state. These iron preparations are also referred to as "iron sugar preparations" since in this case soluble complexes of the trivalent iron are formed with saccharose, dextrans, dextrins, etc., which not only prevent precipitation of iron oxyhydrates in the parenteral preparation but also prevent it specifically when the iron dextran preparation according to the invention is injected or infused into the human or animal body.

DE 938 502 describes a method for producing a colloidal ferrihydroxide dextran complex suitable for the treatment of iron deficiency anaemia, which is primarily used for intramuscular injection but may also be employed intravenously.

DE 12 19 171 likewise describes a method for producing a preparation containing iron for intramuscular injection using ferric salt solutions, oxycarboxylic acids and hexites. Citric acid, gluconic acid, tartaric acid and malic acid are envisaged as oxycarboxylic acids here. Dextrin or a low molecular weight dextran fraction may also be added here.

EP 00 44 050 likewise describes a method for producing iron(III) hydroxide dextran complexes, with an alkali carbonate, ammonium carbonate or a carbonate of an organic base, which is inert with respect to the reaction components, being added to an acidic solution containing a partially depolymerized dextran and an iron(III) salt, an alkali metal hydroxide or ammonium hydroxide subsequently being added, the suspension formed being brought into solution by heating and the solution being processed in a manner known per se.

EP 06 34 174 A1 discloses a preparation for iron deficiency treatment for the veterinary sector, which describes a complex of a carboxylated dextran, obtained by a particular microbiological process, with an iron salt.

The object of the present invention is to provide an alternative iron preparation for parenteral application, which has improved properties compared with the iron preparations of the prior art. For equal physicochemical properties, the improved properties may consist in (i) reduced side effect potential, for example reduced toxicity, (ii) improved bioavailability, (iii) improved environmental friendliness and/or simple production process.

Said objects are achieved by the subject-matters according to the invention, as described in the present description but also in the appended claims.

One subject-matter of the present invention therefore relates to an aqueous iron dextran preparation for the prophylaxis or treatment of iron deficiency states in a human or an animal, the iron being present in the form of Fe³⁺ ions and forming a water-soluble complex with the dextran, characterised in that the preparation comprises one, two, three, four or more compounds of the general formula (I) 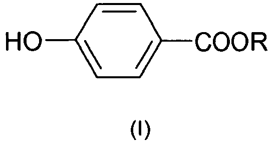 where R represents a C1 to C4 alkyl radical, and/or comprises salts thereof.

Another subject-matter of the present invention relates to a multiple dose preparation for the parenteral prophylaxis or treatment of iron deficiency states in a human or an animal, characterised in that the multiple dose preparation comprises an aqueous iron dextran preparation according to the invention and a suitable container, or consists thereof.

Another subject-matter of the present invention relates to a kit comprising or consisting of
a) a multiple dose preparation according to the invention and
b) one, two, three or more syringes suitable for injecting the preparation or
c) one, two or more infusion sets for infusing the preparation.

Another subject-matter of the present invention relates to a use of an aqueous iron dextran preparation according to the invention for the parenteral prophylaxis or treatment of an iron deficiency state in a human or an animal.

Another subject-matter of the present invention relates to a use of one, two, three, four or more compounds of the general formula (I), 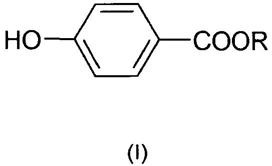 where R represents a C1 to C4 alkyl radical, and/or salts thereof, to produce an iron dextran preparation according to the invention.

Another subject-matter of the present invention relates to a use of one, two, three, four or more compounds of the general formula (I), 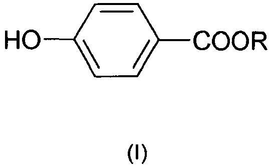 where R represents a C1 to C4 alkyl radical, and/or salts thereof, to reduce the side effects of an iron dextran preparation according to the invention in the case of parenteral application in a human or an animal.

Another subject-matter of the present invention relates to a method for producing an iron dextran preparation according to the invention, characterised in that
a) one or more different Fe³⁺ compounds are provided,
b) one or more different dextran compounds are provided,
c) one, two, three, four or more compounds of the general formula (I) are provided, 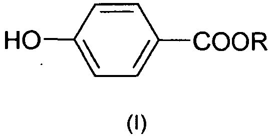 where R represents a C1 to C4 alkyl radical, and/or salts thereof, and
d) the compounds of steps a), b) and c) are dissolved in water.

The present invention is based on the discovery that an iron dextran preparation according to the invention has improved properties compared with the iron preparations of the prior art.

These include in particular improved properties in relation to a reduced side effect potential, since few inflammations have been observed in the case of parenteral application owing to low toxicity. It has furthermore been found that improved bioavailability could be observed compared with preparations from the prior art.

The mechanism which leads to the improvement in bioavailability could not yet be determined with conclusive certainty, although it is assumed that one or more compounds of the formula (I), as described above, contribute to the improvement in bioavailability.

Another advantage of the iron dextran preparation according to the invention is that the proportion of the preservative phenol conventionally used in multiple dose preparations can be reduced by using one or more compounds of the formula (I), as described above, or that phenol as a preservative is entirely replaced by one or more compounds of the formula (I). This is advantageous in particular since phenol is classed as a toxic compound and is therefore less environmentally friendly.

The present invention therefore provides subject-matters that have surprising advantages compared with the subject-matters of the prior art, which were not predictable for a person skilled in the art.

The preferred embodiments of the subject-matters according to the invention will be described below. Preferred embodiments of the individual subject-matters according to the invention may also be preferred in the other subject-matters as well, even if this is not explicitly described.

In the sense of the present invention, the term "C1 to C4 alkyl radicals" includes methyl, ethyl, n- propyl, isopropyl, n-butyl, tert.-butyl.

Preferred compounds of the formula (I) are selected from the group consisting of methyl 4-hydroxybenzoate, ethyl 4-hydroxybenzoate, propyl 4-hydroxybenzoate, butyl 4-hydroxybenzoate and salts thereof. Particularly preferred for use in iron dextran preparations according to the invention are sodium salts of the following combination of compounds (i) methyl 4-hydroxybenzoate preferred within the range of 0.5 to 2.5 mg/ml and propyl 4-hydroxybenzoate preferred within the range of 0.05 to 0.38 mg/ml or (ii) methyl 4-hydroxybenzoate, ethyl 4-hydroxybenzoate, propyl 4-hydroxybenzoate and butyl 4-hydroxybenzoate preferred for each of these compounds independent of the others within the range of 0.3 to 1.0 mg/ml or (iii) ethyl 4-hydroxybenzoate and butyl 4-hydroxybenzoate preferred for each of these compounds independent of the others within the range of 0.5 to 2.0 mg/ml, wherein the proportion of each compound mentioned in this paragraph is related to the total volume of the final preparation. The ranges mentioned in this paragraph can also be valid for other salts than the sodium salts as well as for the respective esters.

"Parenteral", in particular "parenteral preparation", in the sense of the present invention is to be understood as a sterile preparation which is intended for injection (injectables) or infusion (infusibles) into the human or animal body.

Injection preparations in the sense of this invention are preparations which comprise a volume that allows repeated application of from 0.5 to 5 ml, the preparations preferably having volumes of up to 500 ml, more preferably 50 ml, 100 ml or 200 ml.

Infusion preparations in the sense of this invention conventionally have larger volumes than injection preparations, preferably more than 100 ml, more preferably 500 ml.

Preparations according to the invention may possibly need to be adapted to respectively necessary prerequisites for injection preparations, preferably for application into muscular tissue, or infusion preparations preferably for application into vascular veins. In particular, it may be the case that the iron dextran solution according to the invention has to be diluted for intravenous application. In this case all conventional solvents for intravenous application may be used, 5% strength glucose solution or 0.9% strength sterile NaCl solution preferably being used.

The term "multiple dose preparation" according to one of the subject-matters of the present invention is intended to mean an iron dextran preparation according to the invention which is provided in a suitable container, so that its volume is sufficient for two, three, four or more conventional parental applications into the human or animal body, i.e. for multiple use, for the prophylaxis or treatment of iron deficiency states. Since in this case a suitable volume of an iron dextran preparation according to the invention needs to be taken two, three, four or more times from the suitable container, the iron dextran preparation according to the invention must be correspondingly preserved in order to satisfy the requirements of a multiple dose preparation.

Parenteral application of the iron dextran preparation according to the invention and its preferred embodiments is preferably used for pigs, particularly preferably for the prophylaxis of iron deficiency anaemia in young piglets.

An aqueous iron dextran preparation according to the invention may likewise also contain phenol. Phenol is then present in a quantity such that phenol imparts conventional preservative properties. The phenol content preferably lies in a range of from 0 to 10 mg/ml, more preferably 5 mg/ml, expressed in terms of the volume of the final preparation.

An iron dextran preparation according to the invention, however, preferably comprises phenol in a concentration which is conventionally no longer sufficient to impart preservative properties. The preservative property is then imparted by combination with one or more compounds of the general formula (I), as described above.

The phenol content therefore preferably lies in a range of from 0 to 5 mg/ml, more preferably at 0 mg/ml, expressed in terms of the final volume of the iron dextran preparation according to the invention.

In an iron dextran preparation according to the invention, the iron dextran complex conventionally has an average molecular weight (Mn) of from 50,000 to 150,000 daltons. Average molecular weights (Mn) of the iron dextran complex from 90,000 to 120,000 daltons are preferred.

The dextran in the iron dextran complex of an iron dextran preparation according to the invention has an average molecular weight (Mw) conventionally of from 400 to 20,000 daltons. Here, average molecular weights (Mw) of the dextran in an iron dextran preparation according to the invention of from 5000 to 7500 daltons are preferred, or alternatively from 400 to 2500 daltons. The lower the average molecular weight (Mw) of the dextran is, the better its compatibility is.

For an iron dextran preparation according to the invention, all conventional Fe³⁺ salts and oxides may be used, preferably FeCl₃ or Fe₂O₃, with FeCl₃ being particularly preferred for cost and toxicity reasons.

In an iron dextran preparation according to the invention, the proportion of Fe³⁺ by weight expressed in terms of the total volume of the final preparation conventionally lies in the range of 20 to 250 g/l, the proportion of Fe³⁺ by weight having been calculated based on the relative atomic mass 55.847 g/mol of Fe. Proportions of Fe³⁺ by weight, expressed in terms of the total volume of the final iron dextran preparation according to the invention, from 95 to 105 g/l (corresponding approximately to a 10% strength solution), alternatively from 190 to 210 g/l (corresponding approximately to a 20% strength solution) or alternatively from 45 to 55 g/l (corresponding approximately to a 5% strength solution) are preferred here.

The total volume of the final preparation conventionally represents a volume which can be used for a multiple dose application, i.e. for multiple use. The total volume of the final iron dextran preparation according to the invention preferably lies in the range of from 10 to 100 ml, more preferably 100 ml or 250 ml.

The water for the aqueous iron dextran preparation according to the invention is conventionally injection grade water.

An iron dextran preparation according to the invention may also comprise further auxiliaries, which can conventionally be used for producing parenterals, injectables or infusibles. Preferred auxiliaries are selected from the group consisting of: sodium chloride, sodium carbonate, hydrochloric acid, sodium hydroxide, benzyl alcohol and/or chlorocresol, organic acids, preferably ascorbic acid, sodium-EDTA and/or sodium bisulphite.

As already described above, one or more compounds of the formula (I) and their preferred embodiments already impart preservative properties, so that preferably no other preservatives must additionally be included in the preparation according to the invention. As an alternative, however, the preparation according to the invention may also comprise other conventional preservatives, preferably phenol, benzyl alcohol and/or chlorocresol, in concentrations such that they conventionally impart preservative properties, but also in concentrations in which they conventionally impart no preservative properties but lead to preservation of the iron dextran preparation according to the invention in combination with one, two, three, four or more compounds of the formula (I) and their preferred embodiments.

All containers conventionally suitable for multiple use may be used as containers for the multiple dose preparation according to the invention. These containers are preferably selected from the group consisting of: glasses, preferably pharmaceutical glass type 1 and type 2, or plastics/polymers, preferably polypropylene (PP), polyethylene (PE) and polyethylene terephthalate (PET).

Besides a multiple dose preparation according to the invention and its preferred embodiments, 1, 2 or more suitable syringes for parenteral injection of the solution are provided in the kit according to the invention. Those syringes which are conventionally suitable for the parenteral injection of solutions may be used as suitable syringes.

In the method according to the invention for producing an iron dextran preparation according to the invention, the compounds of the constituents a) to c) are brought into solution by customary methods with water which is provided, preferably injection grade water.

If the preparation according to the invention is intended to contain other auxiliaries, as described above, these are added to an already premixed preparation according to requirements.

In an alternative embodiment, the iron dextran complex which is formed may be precipitated by adding isopropanol and the supernatant may subsequently be decanted. The precipitated iron dextran complex is then preferably spray-dried and subsequently redissolved in water.

The subject-matters of the present invention will be represented below by the following examples, the protective scope according to the invention not being restricted to the examples specifically presented. Preferred embodiments may also be seen in the individual components and method steps, which may be combined with the general and preferred subject-matters according to the invention mentioned above.

### Example 1

### Exemplary examples for the production of the iron dextran preparation according to the invention:

### Production method alternative 1:

### 1 Litre contains:

| | | |
|---|---|---|
| 1. | iron dextran powder (30%) | 333.3 g |
| 2. | injection grade water | 860.5 g |
| 3. | Na PHB methyl ester | 0.5 g |
| 4. | Na PHB propyl ester | 0.05 g |
| 5. | sodium chloride | 1.0 g |

HCL or NAOH are optionally used for pH adjustment.

The injection grade water is provided at about 65°C and the additive (NaCl) is dissolved therein. The iron dextran is subsequently added. The PHB ester is then added while stirring. The pH is now adjusted to 11.5 with NaOH. The pH is subsequently adjusted to 6.5 with HCl. Finally, it is made up to the target batch quantity with injection grade water.

### Production method alternative 2:

### 1 Litre contains:

| | | |
|---|---|---|
| 1. | iron dextran solution (15%) | 666.7 g |
| 2. | injection grade water | 527.2 g |
| 3. | Na PHB methyl ester | 0.5 g |
| 4. | Na PHB propyl ester | 0.05 g |
| 5. | sodium chloride | 1.0 g |

HCL and/or NAOH are optionally used for pH adjustment.

The injection grade water is provided at about 65°C and the additive (NaCl) is dissolved therein. The iron dextran solution is subsequently added. The PHB ester is then added while stirring. The pH is now adjusted to 11.5 with NaOH. The pH is subsequently adjusted to 6.5 with HCl. Finally, it is made up to the target batch quantity with injection grade water.

### Example 2

### 1. Test Article Details

### Formulation A (state of the art)

| | |
|---|---|
| Active ingredients: | 330 mg iron(III)-hydroxide-dextran-complex/ml consisting of: |
| | 191 mg iron(III)-hydroxide corresponding to 100 mg iron, |
| | 139 mg dextran (mean molecular weight 6000); |
| | 5 mg phenol/ml |
| | |
| Physical state: | solution for injection |

### Formulation B (according to the invention)

| | |
|---|---|
| Active ingredients: | 303 to 357 mg iron(III)-hydroxide-dextran-powder 28 to 33% / ml corresponding to 100 mg iron, |
| | 1.326 mg methyl-4-hydroxybenzoate sodium, |
| | 0.013 mg propyl-4-hydroxybenzoate sodium |
| Physical state: | solution for injection |

### 2. Experimental Design and Procedures

### Experimental Design

The aim of this exploratory study was to evaluate the efficacy and tolerance of the two Formulations A and B in piglets when administered by a single subcutaneous or intramuscular injection.

Sixteen clinically healthy 2 days old, male and female domestic pigs with a body weight range of 1.0 to 2.9 kg were assigned to one of four test groups of this study, each consisting of 2 male and 2 female animals. All animals received Formulation A (groups I and II) or Formulation B (groups III and IV) once on day 0 either by intramuscular (groups I and III) or subcutaneous (groups II and IV) injection. All injections were given at a dose of 200 mg iron (Fe³⁺)/kg body weight corresponding to 2 ml solution/kg.

Blood samples for the determination of plasma iron concentrations were collected from all animals at 0 h (i.e. prior to treatment) and at 1, 6, 10, 24 and 48 h after treatment. Clinical observations were conducted at these time points. Assessment of the injection sites were performed once daily and body weights were determined prior to treatment on day 0 and at study completion (i.e. on day 2). Animals of groups III and IV were subjected to necropsy on day 2.

### Injection Site Evaluation

The injection sites were evaluated once prior to treatment and once daily afterwards.

Injection site evaluation included assessment of:

### Redness

The injection site was observed for any appearance of redness, which was assessed using the following scores:

| | | |
|---|---|---|
| Score 1 | = none | (no reddening observable) |
| Score 2 | = slight | (reddening of the injection site and the immediate surrounding area) |
| Score 3 | = moderate | (reddening of the injection site and the larger surrounding area) |
| Score 4 | = severe | (dark red or black colouration of the injection site and the surrounding area) |

### Swelling

The injection site was observed and palpated for any swelling, which was assessed using the following scores:

| | | |
|---|---|---|
| Score 1 | = none | (no swelling perceptible) |
| Score 2 | = minimal | (minimal, only palpable swelling at the injection site) |
| Score 3 | = slight | (slight swelling of the injection site and the immediate surrounding area) |
| Score 4 | = moderate | (distinct swelling of the injection site and the surrounding area) |
| Score 5 | = severe | (marked swelling extending far over the injection site) |

### Temperature

The hand was placed over the injection site and the temperature of the overlaying skin was assessed in comparison with that of other body regions. The temperature was recorded using the following scores:

| | | |
|---|---|---|
| Score 1 | = "normal" | (temperature appears "normal", similar to skin temperature of other body regions of the animal) |
| Score 2 | = warm | (temperature appears slightly warmer than the skin temperature of other body regions of the animal) |
| Score 3 | = hot | (temperature appears markedly warmer than the skin temperature of other body regions of the animal) |

### Pain

Pain was assessed by palpation at the injection site using the following scores:

| | | |
|---|---|---|
| Score 1 | = absent | (no signs of pain perceptible) |
| Score 2 | = present | (signs of pain perceptible) |

Any other observed specific findings at the injection site were also recorded.

### Clinical Chemistry - Determination of Plasma Iron Concentrations

Blood samples (approx. 1 ml) were collected in sodium heparin tubes from the V. jugularis/V. cava cranialis for clinical chemistry investigations using a disposable sterile needle (23G, 0.6 x 30 mm).

After withdrawal, the heparinised blood sample were gently agitated to prevent coagulation, put into a container with cooling elements and transported to the testing facility.

Plasma samples were prepared there and analysed for iron concentrations [µmol/l] using a BECKMAN Synchron CX^{®}7 analyser (Beckman Coulter, Inc., Fullterton, USA).

### 3. Evalulation of Data

All individual values are tabulated per animal, treatment group and time point of examination.

For body weights and plasma iron concentrations descriptive statistics were calculated and tabulated.

Plasma concentrations of iron are also graphically presented over time.

The statistical/experimental unit was the individual animal.

**Table 1: Injection Site Evaluation - Group I (Individual findings)**

| Day of the Study | Animal No. | | | | | | | | | | | | | | | |
|---|---|---|---|---|---|---|---|---|---|---|---|---|---|---|---|---|
| | 101 | | | | 102 | | | | 151 | | | | 152 | | | |
| | R | S | T | P | R | S | T | P | R | S | T | P | R | S | T | P |
| 0 | 1* | 1* | 1* | 1* | 1* | 1* | 1* | 1* | 1* | 1* | 1* | 1* | 1* | 1* | 1* | 1* |
| 1 | 1 | 1 | 1 | 1 | 1 | 2 | 1 | 1 | 1 | 2 | 1 | 1 | 1 | 1 | 1 | 1 |
| 2 | 1 | 1 | 1 | 1 | 1 | 2 | 1 | 1 | 1 | 2 | 1 | 1 | 1 | 1 | 1 | 1 |

| | | | | | | | | | | | | | | | | |
|---|---|---|---|---|---|---|---|---|---|---|---|---|---|---|---|---|
| * prior to treatment R = Redness: 1 = none 2 = slight 3 = moderate 4 =severe S = Swelling: 1 = none 2 = minimal 3 = slight 4 = moderate 5 = severe T = Temperature: 1 = "normal" 2 = warm 3 = hot P = Pain: 1 = absent 2 = present | | | | | | | | | | | | | | | | |

**Table 2: Injection Site Evaluation - Group II (Individual findings)**

| Day of the Study | Animal No. | | | | | | | | | | | | | | | |
|---|---|---|---|---|---|---|---|---|---|---|---|---|---|---|---|---|
| | 201 | | | | 202 | | | | 251 | | | | 252 | | | |
| | R | S | T | P | R | S | T | P | R | S | T | P | R | S | T | P |
| 0 | 1* | 1* | 1* | 1* | 1* | 1* | 1* | 1* | 1* | 1* | 1* | 1* | 1* | 1* | 1* | 1* |
| 1 | 1 | 2 | 1 | 1 | 1 | 1 | 1 | 1 | 1 | 2 | 1 | 1 | 1 | 1 | 1 | 1 |
| 2 | 1 | 1 | 1 | 1 | 1 | 1 | 1 | 1 | 1 | 1 | 1 | 1 | 1 | 1 | 1 | 1 |

| | | | | | | | | | | | | | | | | |
|---|---|---|---|---|---|---|---|---|---|---|---|---|---|---|---|---|
| * prior to treatment R = Redness: 1 = none 2 = slight 3 = moderate 4 =severe S = Swelling: 1 = none 2 = minimal 3 = slight 4 = moderate 5 = severe T = Temperature: 1 = "normal" 2 = warm 3 = hot P = Pain: 1 = absent 2 = present | | | | | | | | | | | | | | | | |

**Table 3: Injection Site Evaluation - Group III (Individual findings)**

| Day of the Study | Animal No. | | | | | | | | | | | | | | | |
|---|---|---|---|---|---|---|---|---|---|---|---|---|---|---|---|---|
| | 301 | | | | 302 | | | | 351 | | | | 352 | | | |
| | R | S | T | P | R | S | T | P | R | S | T | P | R | S | T | P |
| 0 | 1* | 1* | 1* | 1* | 1* | 1* | 1* | 1* | 1* | 1* | 1* | 1* | 1* | 1* | 1* | 1* |
| 1 | 1 | 2 | 1 | 1 | 1 | 1 | 1 | 1 | 1 | 1 | 1 | 1 | 1 | 1 | 1 | 1 |
| 2 | 1 | 1 | 1 | 1 | 1 | 1 | 1 | 1 | 1 | 1 | 1 | 1 | 1 | 1 | 1 | 1 |

| | | | | | | | | | | | | | | | | |
|---|---|---|---|---|---|---|---|---|---|---|---|---|---|---|---|---|
| * prior to treatment R = Redness: 1 = none 2 = slight 3 = moderate 4 =severe S = Swelling: 1 = none 2 = minimal 3 = slight 4 = moderate 5 = severe T = Temperature: 1= "normal" 2 = warm 3 = hot P = Pain: 1 = absent 2 = present | | | | | | | | | | | | | | | | |

**Table 4: Injection Site Evaluation - Group IV (Individual findings)**

| Day of the Study | Animal No. | | | | | | | | | | | | | | | |
|---|---|---|---|---|---|---|---|---|---|---|---|---|---|---|---|---|
| | 401 | | | | 402 | | | | 451 | | | | 452 | | | |
| | R | S | T | P | R | S | T | P | R | S | T | P | R | S | T | P |
| 0 | 1* | 1* | 1* | 1* | 1* | 1* | 1* | 1* | 1* | 1* | 1* | 1* | 1* | 1* | 1* | 1* |
| 1 | 1 | 1 | 1 | 1 | 1 | 1 | 1 | 1 | 1 | 1 | 1 | 1 | 1 | 1 | 1 | 1 |
| 2 | 1 | 1 | 1 | 1 | 1 | 1 | 1 | 1 | 1 | 1 | 1 | 1 | 1 | 1 | 1 | 1 |

| | | | | | | | | | | | | | | | | |
|---|---|---|---|---|---|---|---|---|---|---|---|---|---|---|---|---|
| * prior to treatment R = Redness: 1 = none 2 = slight 3 = moderate 4 =severe S = Swelling: 1 = none 2 = minimal 3 = slight 4 = moderate 5 = severe T = Temperature: 1= "normal" 2 = warm 3 = hot P = Pain: 1 = absent 2 = present | | | | | | | | | | | | | | | | |

**Table 5: Injection Site Evaluation - Other Findings - Groups I and II (Individual findings)**

| Day of the Study | Animal No. | | | | | | | |
|---|---|---|---|---|---|---|---|---|
| | 101 | 102 | 151 | 152 | 201 | 202 | 251 | 252 |
| 0 | -* | -* | -* | -* | -* | -* | -* | -* |
| 1 | 1a | 1a | 1a | 1a | 1a | 1a | 1a | 1a |
| 2 | 1a | 1b | 1a | 1b | 1a | 1a | 1a | 1a |

| | | | | | | | | |
|---|---|---|---|---|---|---|---|---|
| * prior to treatment - = no specific findings 1a = dark, diffuse colouration (∼ 1.5 - 4 x 1.5 - 5 cm) 1 b = slightly dark, diffuse colouration (∼ 1.5 - 2 x 1 - 2 cm) | | | | | | | | |

**Table 6: Injection Site Evaluation - Other Findings - Groups III and IV (Individual findings)**

| Day of the Study | Animal No. | | | | | | | |
|---|---|---|---|---|---|---|---|---|
| | 301 | 302 | 351 | 352 | 401 | 402 | 451 | 452 |
| 0 | -* | -* | -* | -* | -* | -* | -* | -* |
| 1 | 1b | - | - | - | - | 1b | 1b | - |
| 2 | -, n | -, n | -, n | -, n | -, n | -, n | -, n | -, n |

| | | | | | | | | |
|---|---|---|---|---|---|---|---|---|
| * prior to treatment - = no specific findings n = necropsy 1b = slightly dark, diffuse colouration (-1.5-2x1-2 cm) | | | | | | | | |

**Table 7 Plasma Iron Concentrations [µmol/l] - Groups I and II (Mean values and standard deviations)**

| Day of the Study | Time Point | Group I | | | Group II | | |
|---|---|---|---|---|---|---|---|
| | | n | Mean | SD | n | Mean | SD |
| 0 | 0 h* | 4 | 26.33 | 20.96 | 4 | 12.98 | 2.89 |
| | 1 h | 4 | 44.23 | 6.85 | 4 | 37.38 | 5.92 |
| | 6 h | 4 | 71.75 | 7.07 | 4 | 67.93 | 13.32 |
| | 10 h | 4 | 79.90 | 8.49 | 4 | 75.43 | 9.13 |
| 1 | 24 h | 4 | 43.50 | 19.83 | 4 | 59.05 | 16.89 |
| 2 | 48 h | 4 | 13.90 | 8.12 | 4 | 31.13 | 10.35 |

| | | | | | | | |
|---|---|---|---|---|---|---|---|
| * prior to treatment | | | | | | | |

**Table 8 Plasma Iron Concentrations [µmol/l] - Groups III and IV (Mean values and standard deviations)**

| Day of the Study | Time Point | Group I | | | Group II | | |
|---|---|---|---|---|---|---|---|
| | | n | Mean | SD | n | Mean | SD |
| 0 | 0 h* | 4 | 10.48 | 6.09 | 4 | 12.20 | 3.75 |
| | 1 h | 4 | 77.58 | 23.22 | 4 | 55.60 | 7.75 |
| | 6 h | 4 | 129.25 | 45.27 | 4 | 112.38 | 5.15 |
| | 10h | 4 | 150.00 | 15.69 | 4 | 125.88 | 18.58 |
| 1 | 24 h | 4 | 97.50 | 16.19 | 4 | 122.00 | 13.58 |
| 2 | 48 h | 4 | 43.65 | 12.34 | 4 | 66.63 | 20.21 |

| | | | | | | | |
|---|---|---|---|---|---|---|---|
| * prior to treatment | | | | | | | |

**Table 9 Individual Plasma Iron Concentrations [µmol/I] - Groups I and II (Individual values)**

| Day of the Study | Time Point | Animal No. | | | | | | | |
|---|---|---|---|---|---|---|---|---|---|
| | | 101 | 102 | 151 | 152 | 201 | 202 | 251 | 252 |
| 0 | 0 h* | 56.0 | 23.4 | 18.9 | 7.0 | 15.6 | 10.0 | 15.3 | 11.0 |
| | 1 h | 53.2 | 40.0 | 37.9 | 45.8 | 34.4 | 44.3 | 39.9 | 30.9 |
| | 6 h | 77.9 | 77.2 | 68.7 | 63.2 | 56.9 | 79.0 | 55.9 | 79.9 |
| | 10 h | 78.0 | 90.0 | 82.0 | 69.6 | 75.7 | 83.0 | 62.5 | 80.5 |
| 1 | 24 h | 31.8 | 71.7 | 42.7 | 27.8 | 42.6 | 75.4 | 46.5 | 71.7 |
| 2 | 48 h | 9.4 | 25.6 | 7.6 | 13.0 | 28.4 | 34.5 | 18.5 | 43.1 |

| | | | | | | | | | |
|---|---|---|---|---|---|---|---|---|---|
| * prior to treatment | | | | | | | | | |

**Table 10 Individual Plasma Iron Concentrations [µmol/l] - Groups III and IV (Individual values)**

| Day of the Study | Time Point | Animal No. | | | | | | | |
|---|---|---|---|---|---|---|---|---|---|
| | | 301 | 302 | 351 | 352 | 401 | 402 | 451 | 452 |
| 0 | 0 h* | 6.8 | 4.0 | 17.0 | 14.1 | 17.5 | 8.7 | 10.9 | 11.7 |
| | 1 h | 59.8 | 75.3 | 64.2 | 111.0 | 46.2 | 52.7 | 59.8 | 63.7 |
| | 6 h | 77.0 | 177.5 | 108.0 | 154.5 | 119.0 | 107.0 | 110.0 | 113.5. |
| | 10 h | 130.0 | 148.5 | 153.5 | 168.0 | 125.5 | 151.0 | 120.5 | 106.5 |
| 1 | 24 h | 79.0 | 118.5 | 96.5 | 96.0 | 130.0 | 137.0 | 110.5 | 110.5 |
| 2 | 48 h | 27.1 | 53.8 | 52.3 | 41.4 | 62.9 | 81.9 | 39.5 | 82.2 |

| | | | | | | | | | |
|---|---|---|---|---|---|---|---|---|---|
| * prior to treatment | | | | | | | | | |

### Injection Site Evaluation

Individual findings at the injection site of the animals of the scored parameters redness, swelling, temperature and pain during the entire period of the study are presented in Tables 1 to 4. A description of other individual findings at the injection site is given in Tables 5 (groups I and II) and 6 (groups III and IV).

Redness was scored on a scale from 1 -4, swelling on a scale from 1 -5, temperature on a scale from 1-3andpainonascalefrom 1-2.

On day 0 (i.e. prior to the treatment), all animals were categorised into score 1 for all scored parameters, corresponding to no redness, no swelling, "normal" temperature and no pain.

After intramuscular administration to group I (Formulation A) and III (Formulation B) animals on day 0, a slight swelling was found at the injection site on day 1 in two of 4 group I animals (i.e. animal nos. 102 and 151) and in one of 4 group III animals (i.e. animal no. 301). On day 2, no swelling was observed in all group III animals but still in animal nos. 102 and 151 of group I.

A dark and diffuse colouration of the injection site area was noted in all group I animals on day 1 in contrast to an only slightly dark and diffuse colouration in only one animal of group III (i.e. animal no. 301). On day 2, a dark or slightly dark and diffuse colouration was still found in all group I animals but not in the animals of group III which received Formulation B.

After subcutaneous administration to group II (Formulation A) and IV (Formulation B) animals on day 0, a slight swelling was found at the injection site on day 1 in two of 4 group II animals (i.e. animal nos. 201 and 251) but in none of the 4 group IV animals. On day 2, all animals of both groups II and IV did not show any swelling anymore.

All group II animals revealed a dark and diffuse colouration of the injection site area on days 1 and 2 whereas a similar finding but of a more slight degree was observed in two of 4 group IV animals (i.e. animal nos. 402 and 451) on day 1 only. On day 2, such a finding could not be found in any animal of group IV having been treated with Formulation B.

No redness, elevated temperature or pain was noted in any animal of all groups at any time after intramuscular or subcutaneous treatment.

In summary, these results indicate that slight swelling and a diffuse dark colouration at the injection site was found at a higher incidence after both, subcutaneous and intramuscular treatment with Formulation A in comparison with Formulation B.

### Clinical Chemistry - Determination of Plasma Iron Concentrations

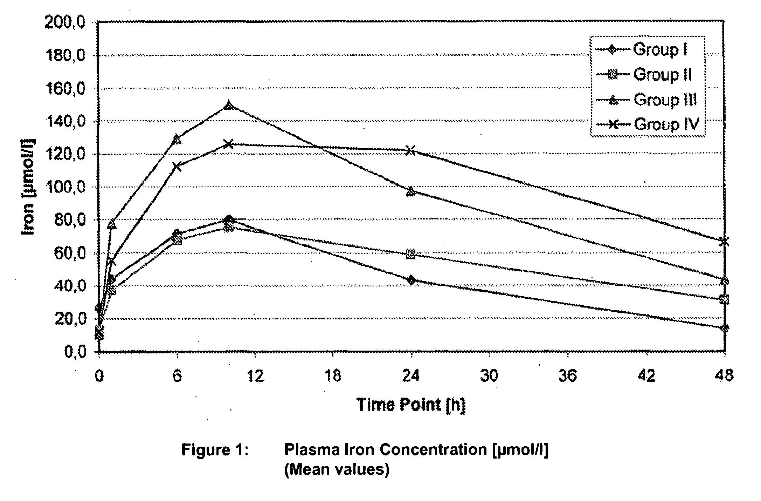

Mean values and standard deviations at the various time points of measurement are presented in Tables 7 (groups I and II) and 8 (groups III and IV), individual values are given in Tables 9 (groups I and II) and 10 (groups III and IV). The mean values course of the plasma iron concentrations over time is graphically presented in Figure 1.

Mean iron concentrations in plasma were 26.33, 12.98, 10.48 and 12.20 µmol/l in groups I, II, III and IV, respectively, before treatment on day 0 (i.e. at 0 h). In group I, individual values varied to a higher extent than in the other three groups.

After intramuscular administration to groups I (Formulation A) and III (Formulation B), mean values increased until 10 h after treatment in both groups and decreased again afterwards. However, the mean maximal plasma concentration was 79.90 µmol/l in group I in contrast to 150.00 umol/l in group III. At 48 h, iron concentrations returned to roughly the pre-treatment levels or even below in group I but were still higher in all animals of group III. The respective mean values were 13.90 and 43.65 umol/l.

A similar result was noted after subcutaneous administration to groups II (Formulation A) and IV (Formulation B). Maximal mean plasma concentrations were found at 10 h after treatment in both groups with values of 75.43 µmol/l in group II and 125.88 µmol/l in group IV the latter being also clearly higher. At 24 h mean values started to decrease again in group II with an actual value of 59.05 µmol/l but remained at roughly the same level in group IV (i.e.122.00 µmol/l).At48 h, iron concentrations decreased in both groups to respective mean values of 31.13 and 66.63 µmol/l but were still higher than pre-treatment levels.

In summary, higher mean concentrations were noted for both formulations at 48 h after subcutaneous injection in comparison with intramuscular injection.

For both routes of administration higher mean plasma concentrations were found at all time points after treatment with Formulation B compared to Formulation A.

### 4. Conclusion

The results of the present example permit the following conclusions:

The test articles Formulation A and Formulation B were generally well tolerated in 2 days old piglets following a single intramuscular or subcutaneous injection at a dose of 200 mg iron (Fe³⁺)/kg body weight.

Slight swelling and a diffuse dark colouration at the injection site was present at a higher incidence after both, subcutaneous and intramuscular injection of Formulation A in comparison with Formulation B.

For both routes of administration (i.e. intramuscular and subcutaneous) higher mean plasma iron concentrations were found at all time points after treatment with Formulation B compared to Formulation A.

## Claims

1. Aqueous iron dextran preparation capable for the prophylaxis or treatment of iron deficiency states in a human or an animal, the iron being present in the form of Fe³⁺ ions and forming a water-soluble complex with the dextran, **characterised in that** the preparation comprises one, two, three, four or more compounds of the general formula (I) 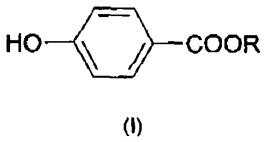 where R represents a C1-C4 alkyl radical, and/or comprises salts thereof.

2. Preparation according to claim 1 for use as medicament.

3. Preparation according to claim 1 or 2 for the prophylaxis or treatment of iron deficiency states in a human or an animal.

4. Preparation according to any one of the preceding claims, **characterised in that** the or one of the compounds of the general formula (I) is selected from the group consisting of methyl 4-hydroxybenzoate, ethyl 4-hydroxybenzoate, propyl 4-hydroxybenzoate, butyl 4-hydroxybenzoate and salts thereof and preferably **characterised in that** the preparation comprises the sodium salts of the following combination of compounds (i) methyl 4-hydroxybenzoate and propyl 4-hydroxybenzoate or (ii) methyl 4-hydroxybenzoate, ethyl 4-hydroxybenzoate, propyl 4-hydroxybenzoate and butyl 4-hydroxybenzoate or (iii) ethyl 4-hydroxybenzoate and butyl 4-hydroxybenzoate.

5. Preparation according to any one of the preceding claims, **characterised in that** the preparation additionally comprises phenol.

6. Preparation according to any one of the preceding claims, **characterised in that** the iron dextran complex has an average molecular weight (Mn) of from 50,000 to 150,000 daltons.

7. Preparation according to any one of the preceding claims, **characterised in that** the dextran in the iron dextran complex has an average molecular weight (Mw) of from 400 to 20,000 daltons.

8. Preparation according to any one of the preceding claims, **characterised in that** the proportion of Fe³⁺ by weight expressed in terms of the total volume of the final preparation lies in the range of 20 to 250 g/l.

9. Preparation according to any one of the preceding claims, **characterised in that** the total volume of the final preparation lies in the range of from 10 to 250 ml.

10. Multiple dose preparation for the parenteral prophylaxis or treatment of iron deficiency states in a human or an animal, **characterised in that** the multiple dose preparation comprises a preparation according to any one of the preceding claims and a suitable container, or consists thereof.

11. Kit comprising or consisting of
a) a multiple dose preparation according to Claim 10 and
b) one, two or more syringes suitable for injecting the solution or
c) one, two or more infusion sets for infusing the preparation.

12. Use of a preparation according to any one of Claims 1 to 9 for the preparation of a medicament for the parenteral prophylaxis or treatment of an iron deficiency state in a human or an animal.

13. Use of one, two, three, four or more compounds of the general formula (I) 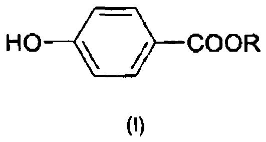 where R represents a C1-C4 alkyl radical, and/or salts thereof, to produce an iron dextran preparation according to any one of Claims 1 to 9.

14. Use of one, two, three, four or more compounds of the general formula (I) 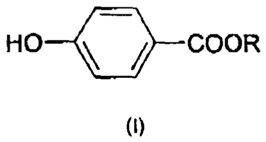 where R represents a C1-C4 alkyl radical, and/or salts thereof, to reduce the side effects of an iron dextran preparation according to any one of Claims 1 to 9 in the case of parenteral application in a human or an animal.

15. Method for producing a preparation according to any one of Claims 1 to 9, **characterised in that**
a) one or more Fe³⁺ compounds are provided,
b) one or more dextran compounds are provided,
c) one, two, three, four or more compounds of the general formula (I) are provided, 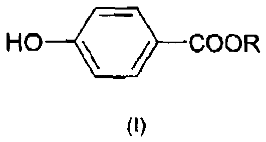 where R represents a C1-C4 alkyl radical, and/or salts thereof, and
d) the compounds of steps a), b) and c) are dissolved in water, preferably in injection grade water.

## Patentansprüche

1. Wässriges Eisendextranpräparat, das der Prophylaxe oder Behandlung von Eisenmangelzuständen bei einem Menschen oder Tier fähig ist, wobei das Eisen in Form von Fe³⁺-Ionen vorliegt und einen wasserlöslichen Komplex mit dem Dextran bildet, **dadurch gekennzeichnet, dass** das Präparat ein, zwei, drei, vier oder mehr Verbindungen der allgemeinen Formel (I) 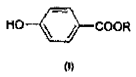 wobei R ein C1-C4-Alkylradikal darstellt, und/oder Salze davon umfasst.

2. Präparat nach Anspruch 1 zur Verwendung als Medikament.

3. Präparat nach Anspruch 1 oder 2 zur Prophylaxe oder Behandlung von Eisenmangelzuständen bei einem Menschen oder einem Tier.

4. Präparat nach einem der vorhergehenden Ansprüche, **dadurch gekennzeichnet, dass** die oder eine der Verbindungen der allgemeinen Formel (I) aus der Gruppe ausgewählt sind/ist bestehend aus Methyl-4-hydroxybenzoat, Ethyl-4-hydroxybenzoat, Propyl-4-hydroxybenzoat, Butyl-4-hydroxybenzoat und Salzen davon und bevorzugt **dadurch gekennzeichnet** sind/ist, dass das Präparat die Natriumsalze der folgenden Kombination von Verbindungen umfasst (i) Methyl-4-hydroxybenzoat und Propyl-4-hydroxybenzoat oder (ii) Methyl-4-hydroxybenzoat, Ethyl-4-hydroxybenzoat, Propyl-4-hydroxybenzoat und Butyl-4-hydroxybenzoat oder (iii) Ethyl-4-hydroxybenzoat und Butyl-4-hydroxybenzoat.

5. Präparat nach einem der vorhergehenden Ansprüche, **dadurch gekennzeichnet, dass** das Präparat zusätzlich Phenol umfasst.

6. Präparat nach einem der vorhergehenden Ansprüche, **dadurch gekennzeichnet, dass** der Eisendextrankomplex ein durchschnittliches Molekulargewicht (Mn) von 50.000 bis 150.000 Dalton aufweist.

7. Präparat nach einem der vorhergehenden Ansprüche, **dadurch gekennzeichnet, dass** das Dextran in dem Eisendextrankomplex ein durchschnittliches Molekulargewicht (Mw) von 400 bis 20.000 Dalton aufweist.

8. Präparat nach einem der vorhergehenden Ansprüche, **dadurch gekennzeichnet, dass** der Gewichtsanteil an Fe3+, als Gesamtvolumen des endgültigen Präparat ausgedrückt, im Bereich von 20 bis 250 g/l liegt.

9. Präparat nach einem der vorhergehenden Ansprüche, **dadurch gekennzeichnet, dass** das Gesamtvolumen des endgültigen Präparats im Bereich von 10 bis 250 ml liegt.

10. Mehrfachdosispräparat für die parenterale Prophylaxe oder Behandlung von Eisenmangelzuständen bei einem Menschen oder einem Tier, **dadurch gekennzeichnet, dass** das Mehrfachdosenpräparat ein Präparat nach einem der vorhergehenden Ansprüche und einen geeigneten Behälter umfasst oder daraus besteht.

11. Kit umfassend oder bestehend aus
a) einem Mehrfachdosenpräparat nach Anspruch 10 und
b) einer, zwei oder mehr Spritzen, die zur Injektion der Lösung geeignet sind oder
c) einer, zwei oder mehr Infusionsbestecken zur Infusion des Präparats.

12. Verwendung eines Präparats nach einem der Ansprüche 1 bis 9 zur Herstellung eines Medikaments für die parenterale Prophylaxe oder Behandlung eines Eisenmangelzustands bei einem Menschen oder einem Tier.

13. Verwendung von einer, zwei, drei, vier oder mehr Verbindungen der allgemeinen Formel (I) 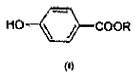 wobei R ein C1-C4-Alkylradikal umfasst, und/oder von Salzen davon zum Herstellen eines Eisendextranpräparats nach einem der Ansprüche 1 bis 9.

14. Verwendung von einer, zwei, drei, vier oder mehr Verbindung der allgemeinen Formel (I) 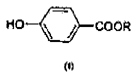 wobei R ein C1-C4-Alkylradikal umfasst, und/oder von Salzen davon zum Reduziere der Nebenwirkungen eines Eisendextranpräparats nach einem der Ansprüche 1 bis 9 im Falle der parenteralen Anwendung bei einem Menschen oder einem Tier.

15. Verfahren zur Herstellung eines Präparats nach einem der Ansprüche 1 bis 9, **dadurch gekennzeichnet, dass**
a) eine oder mehr Fe³⁺-Verbindungen bereitgestellt werden,
b) eine oder mehr Dextranverbindungen bereitgestellt werden,
c) eine, zwei, drei, vier oder mehr Verbindungen der allgemeinen Formel (I), 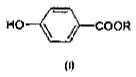 wobei R ein C1-C4-Alkylradikal darstellt, und/oder Salze davon bereitgestellt werden und
d) die Verbindungen aus den Schritten a), b) und c) in Wasser, bevorzugt in Wasser von Injektionsqualität, gelöst werden.

## Revendications

1. Préparation aqueuse de fer dextrane destinée à la prophylaxie ou au traitement d'états de carence en fer chez un humain ou un animal, le fer étant présent sous la forme d'ions Fe³⁺ et formant un complexe soluble dans l'eau avec le dextrane, **caractérisée en ce que** la préparation comprend un, deux, trois, quatre composés ou plus de formule générale (I) 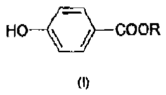 où R représente un radical alkyle en C₁ à C₄ et/ou comprend des sels de celui-ci.

2. Préparation selon la revendication 1, à utiliser comme médicament.

3. Préparation selon la revendication 1 ou 2, pour la prophylaxie ou le traitement d'états de carence en fer chez un humain ou un animal.

4. Préparation selon l'une quelconque des revendications précédentes, **caractérisée en ce que** le ou un des composés de formule générale (I) est choisi dans le groupe consistant en le 4-hydroxybenzoate de méthyle, le 4-hydroxybenzoate d'éthyle, le 4-hydroxybenzoate de propyle, le 4-hydroxybenzoate de butyle et leurs sels et de préférence **caractérisée en ce que** la préparation comprend les sels de sodium de la combinaison suivante de composés (i) 4-hydroxybenzoate de méthyle et - hydroxybenzoate de propyle ou (ii) 4-hydroxybenzoate de méthyle, 4-hydroxybenzoate d'éthyle, 4-hydroxybenzoate de propyle et 4-hydroxybenzoate de butyle ou (iii) 4-hydroxybenzoate d'éthyle et 4-hydroxybenzoate de butyle.

5. Préparation selon l'une quelconque des revendications précédentes, **caractérisée en ce que** la préparation comprend en outre du phénol.

6. Préparation selon l'une quelconque des revendications précédentes, **caractérisée en ce que** le complexe de fer dextrane a une masse moléculaire moyenne (Mn) de 50 000 à 150 000 daltons.

7. Préparation selon l'une quelconque des revendications précédentes, **caractérisée en ce que** le dextrane dans le complexe de fer dextrane a une masse moléculaire moyenne (Mw) de 400 à 20 000 daltons.

8. Préparation selon l'une quelconque des revendications précédentes, **caractérisée en ce que** la proportion de Fe³⁺ en poids exprimée en termes de volume total de la préparation finale se trouve dans la plage de 20 à 250 g/L.

9. Préparation selon l'une quelconque des revendications précédentes, **caractérisée en ce que** le volume total de la préparation finale se situe dans la plage de 10 à 250 mL.

10. préparation à doses multiples destinée à la prophylaxie ou au traitement parentéral d'états de carence en fer chez un humain ou un animal, **caractérisée en ce que** la préparation à doses multiples comprend ou consiste en une préparation selon l'une quelconque des revendications précédentes et un contenant approprié.

11. Nécessaire comprenant ou consistant en
a) une préparation à doses multiples selon la revendication 10 et
b) une, deux seringues ou plus appropriées pour injecter la solution ou
c) un, deux jeux d'infusions ou plus pour infuser la préparation.

12. Utilisation d'une préparation selon l'une quelconque des revendications 1 à 9 pour la préparation d'un médicament destiné à la prophylaxie ou au traitement parentéral d'un état de carence en fer chez un humain ou un animal.

13. Utilisation d'un, de deux, de trois, de quatre composés ou plus de formule générale (I) 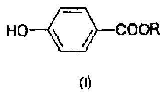 où R représente un radical alkyle en C₁ à C₄ et/ou des sels de celui-ci, pour produire une préparation de fer dextrane selon l'une quelconque des revendications 1 à 9.

14. Utilisation d'un, de deux, de trois, de quatre composés ou plus de formule générale (I) 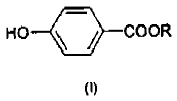 où R représente un radical alkyle en C₁ à C₄ et/ou des sels de celui-ci, pour réduire les effets secondaires d'une préparation de fer dextrane selon l'une quelconque des revendications 1 à 9, dans le cas d'une application parentérale chez un humain ou un animal.

15. Procédé de production d'une préparation selon l'une quelconque des revendications 1 à 9, **caractérisé en ce que**
a) un ou plusieurs composés de Fe³⁺ sont fournis,
b) un ou plusieurs composés de dextrane sont fournis,
c) un, deux, trois, quatre composés ou plus de formule générale (1) sont fournis, 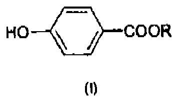 où R représente un radical alkyle en C₁ à C₄ et/ou des sels de celui-ci, et
d) les composés des étapes a), b) et c) sont dissous dans l'eau, de préférence dans de l'eau de qualité pour injection.
